(19) Europäisches Patentamt
European Patent Office
Office européen des brevets

(11) **EP 1 992 328 A1**

(12) **EUROPEAN PATENT APPLICATION**
published in accordance with Art. 158(3) EPC

(43) Date of publication:
**19.11.2008 Bulletin 2008/47**

(21) Application number: **07707947.3**

(22) Date of filing: **02.02.2007**

(51) Int Cl.:
*A61K 8/37* (2006.01)       *A61K 8/06* (2006.01)
*A61K 8/40* (2006.01)       *A61K 8/891* (2006.01)
*A61K 8/894* (2006.01)       *A61Q 17/04* (2006.01)

(86) International application number:
**PCT/JP2007/051787**

(87) International publication number:
**WO 2007/091490 (16.08.2007 Gazette 2007/33)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.02.2006 JP 2006028874**

(71) Applicant: **Shiseido Company, Limited**
**Chuo-ku**
**Tokyo 104-8010 (JP)**

(72) Inventor: **TAKAKURA, Yoshihito**
**Yokohama-shi, Kanagawa 2248558 (JP)**

(74) Representative: **Henkel, Feiler & Hänzel**
**Patentanwälte**
**Maximiliansplatz 21**
**80333 München (DE)**

(54) **WATER-IN-OIL EMULSION TYPE SUNSCREEN PREPARATION**

(57)   An object of the present invention is to provide a water-in-oil type emulsion sunscreen cosmetic which can achieve excellent UV protection capability, excellent usability such as good adaptation to the skin and non-stickiness, excellent emulsion stability and excellent water resistance.

   The water-in-oil type emulsion sunscreen cosmetic in the present invention is **characterized by** comprising (a) 0.2 to 14 % by mass of methoxycinnamate ester, (b) 0.02 to 14 % by mass of octocrylene, (c) 0.2 to 14 % by mass of dimethylpolysiloxane, (d) 0.02 to 14 % by mass of monoester oil represented by the formula: $R_1COOR_2$ wherein $R_1$ represents an alkyl group having 5 to 11 carbon atoms and $R_2$ represents an alkyl group having 3 to 11 carbon atoms, and (e) 0.02 to 6 % by mass of silicone-chain branched-type alkyl/polyoxyethylene-modified silicone.

EP 1 992 328 A1

**Description**

RELATED APPLICATIONS

This application claims the priority of Japanese Patent Application No. 2006-028874 filed on February 6, 2006, which are incorporated herein by reference.

FIELD OF THE INVENTION

The present invention relates to water-in-oil type emulsion sunscreen cosmetics. In particular, the present invention relates to water-in-oil type emulsion sunscreen cosmetics that have excellent UV protection capability, water resistance, usability, and emulsion stability.

BACKGROUND OF THE INVENTION

Generally, in order to block UV radiation to the skin and achieve high SPF (Sun Protection Factor) values, UV absorbers (refer to patent literature 1, for example) or UV scatterer (zinc oxide, titanium dioxide etc.) are blended in sunscreen cosmetics.
UV absorbers, which shield UV radiation by absorbing light energy, is generally highly polar oil which has an undesirable property in a safety for the skin such as dermal irritancy. Therefore, UV absorbers are used in combination with such as silicone oil which is free from irritating properties and has good usability. However, when UV absorber, which is highly polar oil, and silicone oil, which is non-polar oil, are used together, oil phase becomes less homogeneous, which causes problems in the emulsion stability or problems of uneven coating on the skin. Particularly, a combination use of methoxycinnamate ester and octocrylene as UV absorbers achieves excellent UV shielding effects, however, it has significantly low emulsion stability in the system of water-in-oil type emulsion, which causes problems in productization.

[Patent literature 1] Japanese Unexamined Patent Publication No. H9-151108.

DISCLOSURE OF THE INVENTION

PROBLEM TO BE SOLVED BY THE INVENTION

The object of the present invention is to provide water-in-oil type emulsion sunscreen cosmetics that achieve an excellent UV shielding effect, an excellent usability such as good adaptation to the skin and non-stickiness, an excellent emulsion stability, and an excellent water resistance.

MEANS TO SOLVE THE PROBLEM

The present inventors have diligently studied to solve the above-described problem. As a result, the present inventors have found that water-in-oil type emulsion sunscreen cosmetics with an excellent UV shielding effect, usability, water resistance and emulsion stability could be achieved by blending a specific ester oil and a specific silicone emulsifier in the system which methoxycinnamate ester and octocrylene are used together as UV absorbers and silicone oil is blended, thus leading to completion of the present invention.
The present invention provides water-in-oil type emulsion sunscreen cosmetics comprising (a) 0.2 to 14 % by mass of methoxycinnamate ester, (b) 0.02 to 14 % by mass of octocrylene, (c) 0.2 to 14 % by mass of dimethylpolysiloxane, (d) 0.02 to 14 % by mass of monoester oil represented by the formula: $R_1COOR_2$ wherein $R_1$ represents an alkyl group having 5 to 11 carbon atoms and $R_2$ represents an alkyl group having 3 to 11 carbon atoms, and (e) 0.02 to 6 % by mass of silicone-chain branched-type alkyl/polyoxyethylene-modified silicone.
The present invention provides the above-described water-in-oil type emulsion sunscreen cosmetics wherein component (a) is ethylhexyl methoxycinnamate.
The present invention provides the above-described water-in-oil type emulsion sunscreen cosmetics wherein component (d) is an ester formed by isocarboxylic acid and isoalcohol and each $R_1$ and $R_2$ have 7 to 10 carbon atoms.

EFFECT OF THE INVENTION

According to the present invention, water-in-oil type emulsion sunscreen cosmetics that can satisfactorily achieve an excellent UV blocking effect and have an excellent usability such as good adaptation to the skin and non-stickiness, an excellent water resistance, and an excellent emulsion stability are provided.

BEST MODE FOR CARRYING OUT THE INVENTION

Hereinafter, the water-in-oil type emulsion sunscreen cosmetics of the present invention will be described in detail.

Methoxycinnamate ester, which is component (a) used in the present invention, is a UV protection agent and is not particularly restricted as long as it can be generally used for cosmetics or phermaceauticals. Methoxycinnamate ester includes ethylhexyl methoxycinnamate such as "Parsol MCX" (DSM Nutrition Japan K.K.), isopropyl methoxycinnamate, isoamyl methoxycinnamate such as "Neo Heliopan E1000" (Haarmann and Reimer), and so on. Particularly, ethylhexyl methoxycinnamate (=octyl methoxy cinnamate) is preferably used in the present invention. One or more component (a) can be used.

The blending amount of component (a) is 0.2 to 14 % by mass with respect to the cosmetics of the present invention, preferably 3 to 10 % by mass, more preferably 5 to 8 % by mass. If the blending amount is less than 0.2 % by mass, a satisfactory UV protection capability cannot be achieved. On the other hand, if the blending amount exceeds 14 % by mass, high polarization of oil phase worsens the emulsion stability, and increase of the amount of residual oil worsens the usability and the water resistance.

Octocrylene (another name: 2-cyano-3,3-diphenyl-2-propenoic acid 2-ethylhexyl ester), which is component (b), is a UV protection agent, and it is commercially available, for example, "Uvinul N539" (BASF) or "Parsol 340" (DSM Nutrition Japan K.K.), and they can be used desirably.

The blending amount of component (b) is 0.02 to 14 % by mass with respect to the cosmetics of the present invention, preferably 0.2 to 7 % by mass, more preferably 1 to 5 % by mass. If the blending amount is less than 0.02 % by mass, a satisfactory UV protection capability cannot be achieved. On the other hand, if the blending amount exceeds 14 % by mass, high polarization of oil phase worsens the emulsion stability, and increase of the amount of residual oil worsens the usability and the water resistance.

Since there is a synergistic effect in a UV protection capability between component (a) and component (b), it is preferred that the blending amount of component (b) is at least 10 % by mass or more with respect to the blending amount of component (a).

Dimethylpolysiloxane, which is component (c), is chained silicone oil with 5000 mm$^2$/s or less viscosity, and is commercially available, for example, "SH200 series" (manufactured by Toray Dow Corning Silicone Co., Ltd.) or "KF 96 series" (manufactured by Shin-Etsu Chemical Co., Ltd.), and they can be used desirably. The thicker the viscosity of dimethylpolysiloxane is, the better the water resistance becomes, but at the same time, the worse the usability and stability become. One or more of dimethylpolysiloxanes may be combined and blended depending on the required water resistance, usability and stability. Particularly, dimethylpolysiloxane with 3 to 10 mm$^2$/s viscosity is preferably used in the present invention.

The blending amount of component (c) is 0.2 to 14 % by mass with respect to the cosmetics of the present invention, preferably 1 to 10 % by mass, more preferably 3 to 7 % by mass. If the blending amount is less than 0.2 % by mass, a satisfactory water resistance and a satisfactory adaptation to the skin cannot be achieved. On the other hand, if the blending amount exceeds 14 % by mass, lost of compatibility of the oil phase worsens emulsion stability.

Branched monoester oil, which is component (d), is represented by the formula: $R_1COOR_2$ wherein $R_1$ represents an alkyl group having 5 to 11 carbon atoms and $R_2$ represents an alkyl group having 3 to 11 carbon atoms. Branched monoester oil, for example, includes isononyl isononanoate, isodecyl isononanoate, isotridecyl isononanoate, 2-ethylhexyl isononanoate, ethylhexyl ethylhexanoate, isononyl ethylhexanoate, cetearyl isononanoate, octyl isononanoate, octyl octanoate, ethylhexyl octanoate and so on. However, the branched monoester oil is not limited by these examples. Particularly, isononyl isononanoate is preferably used in the present invention. One or more component (d) can be used.

The blending amount of component (d) is 0.02 to 14 % by mass with respect to the cosmetics of the present invention, preferably 0.2 to 10 % by mass, more preferably 1 to 7 % by mass. If the blending amount is less than 0.02 % by mass, a satisfactory emulsion stability cannot be achieved. On the other hand, if the blending amount exceeds 14 % by mass, increase of the amount of the residual oil worsens usability and water resistance.

Silicone-chain branched-type alkyl/polyoxyethylene (hereinafter also described as "POE") -modified silicone, which is component (e), is preferably used because of its excellent emulsifying property in both silicone oil and polar oil which are often used in sunscreen cosmetics. One or more component (e) can be used.

The above-described silicone-chain branched-type alkyl/POE-modified silicone is commercially available such as lauryl PEG-9 polydimethylsiloxyethyl dimethicone ("KF-6038" manufactured by Shin-Etsu Chemical Co., Ltd.).

The blending amount of component (e) is 0.02 to 6 % by mass with respect to the cosmetics of the present invention, preferably 0.1 to 3 % by mass, more preferably 0.3 to 2 % by mass. If the blending amount is less than 0.02 % by mass, a satisfactory emulsion stability cannot be achieved. On the other hand, if the blending amount exceeds 6 % by mass, the usability such as stickiness becomes worsen, and water resistance becomes worsen because component (e) tends to re-emulsify.

In the cosmetics of the present invention, in addition to the above-described components, other components normally be used in cosmetics can be blended as necessary so far as the objectives and effects of the present invention are not undermined. Examples of these components include water-soluble polymers, oil-soluble polymers, polymer powders, emulsifying agents (other than the above-described component (e)), waxes, alcohols, hydrocarbon oils, silicon oils (other than the above-described component (c)), fatty acids, higher alcohols, fatty acid esters, drugs, UV absorbers (other than the above-described components (a) and (b)), UV scatterers, and organic-modified clay minerals. However, the components are not limited by these examples.

Examples of water-soluble polymers include a homopolymer and copolymers of 2-acrylamido-2-methyl propane sulfonic acid (hereinafter abbreviated as "AMPS"). The copolymer comprises comonomers such as vinylpyrrolidone, acrylamide, sodium acrylate, and hydroxyethyl acrylate. Examples include AMPS homopolymer, vinylpyrrolidone/AMPS copolymer, dimethylacrylamide/AMPS copolymer, acrylamide/AMPS copolymer, and sodium acrylate/AMPS copolymer.

Further examples include carboxyvinyl polymer, ammonium polyacrylate, sodium polyacrylate, sodium acrylate/ alkyl acrylate/sodium methacrylate/alkyl methacrylate copolymer, carrageenan, pectin, mannan, curdlan, chondroitin sulfate, starch, glycogen, gum arabic, sodium hyaluronate, tragacanth gum, xanthan gum, mucoitin sulfate, hydroxyethyl guar gum, carboxymethyl guar gum, guar gum, dextran, kerato sulfate, locust bean gum, succinoglucan, chitin, chitosan, carboxymethyl chitin, and agar.

Examples of oil-soluble polymers include trimethylsiloxysilicate, alkyl-modified silicone, polyamide-modified silicone.

Examples of polymer powders include dimethicone crosspolymer, (dimethicone/vinyldimethicone) crosspolymer, polymethylsilsesquioxane and so on.

Examples of emulsifying agents (other than the above-described component (e)) include POE/methylpolysiloxane copolymer, silicone-chain branched-type POE/methylpolysiloxane copolymer, straight chain-type alkyl/POE-modified methylpolysiloxane copolymer, crosslinked-type POE/methylpolysiloxane copolymer, glycerin fatty acid ester, polyglycerin fatty acid ester, polyoxyethylene glycerin fatty acid ester, sorbitan fatty acid ester, and polyoxyethylene sorbitan fatty acid ester.

Examples of waxes include beeswax, candelilla wax, carnauba wax, lanolin, lanolin oil, and jojoba wax.

Examples of alcohols include lower alcohols such as ethanol and isopropanol; higher alcohols such as isostearyl alcohol, octyldodecanol, and hexyldecanol; and polyhydric alcohols such as ethylene glycol, propylene glycol, 1,3-butylene glycol, dipropylene glycol, and polybutylene glycol.

Examples of hydrocarbon oils include liquid paraffin, ozokerite, squalane, pristane, paraffin, ceresin, squalene, petrolatum, microcrystalline wax, polyethylene wax, and Fischer-Tropsch wax.

Examples of silicon oils (other than the above-described component (c)), include octamethylsiloxane, decamethyltetrasiloxane, methylhydrogenpolysiloxane, methylphenylpolysiloxane, hexamethylcyclotrisiloxane, octamethylcyclotetrasiloxane, and decamethylcyclopentasiloxane.

Examples of fatty acids include lauric acid, myristic acid, palmitic acid, stearic acid, behenic acid, and arachidonic acid.

Examples of higher alcohols include lauryl alcohol, myristyl alcohol, cetyl alcohol, stearyl alcohol, behenyl alcohol, arachyl alcohol, batyl alcohol, chimyl alcohol, carnaubyl alcohol, ceryl alcohol, koryanyl alcohol, myricyl alcohol, lacceryl alcohol, elaidyl alcohol, isostearyl glyceryl ether, octyl alcohol, triancotyl alcohol, cerakyl alcohol, cetostearyl alcohol, oleyl alcohol, lanolin alcohol, hydrogenated lanolin alcohol, hexyldecanol, and octyldecanol.

Examples of fatty acid esters include myristyl myristate, cetyl palmitate, cholesteryl stearate, and 2-octyldodecyl beeswax fatty acid.

Examples of drugs include L-ascorbic acid and its salt derivatives, glycyrrhizic acid and its derivatives such as dipotassium glycyrrhizate and monoammonium glycyrrhizate, glycyrrhetinic acid and its derivatives such as stearyl glycyrrhetinate, allantoin, tranexamic acid and its salt derivatives, alkoxysalicylic acid and its salt derivatives, glutathione and its salt derivatives, allantoin, and azulene.

Examples of UV absorbers (other than the above-described components (a) and (b)) include PABA derivatives such as para-aminobenzoic acid (hereinafter abbreviated as "PABA"), ethyl PABA, ethyl dihydroxypropyl PABA, ethylhexyl dimethyl PABA, and glyceryl PABA; salicylic acid derivatives such as homosalate, ethylhexyl salicylate, dipropylene glycol salicylate, and TEA salicylate; benzophenone derivatives such as benzophenone-1, benzophenone-2, benzophenone-3 or oxybenzone, benzophenone-4, benzophenone-5, benzophenone-6, benzophenone-8, benzophenone-9, and benzophenone-12; benzylidene camphor derivatives such as 3-benzylidene camphor, 4-methylbenzylidene camphor, benzylidene camphor sulfonic acid, camphor benzalkonium methosulfate, terephthalylidene dicamphor sulfonic acid, and polyacrylamidomethyl benzylidene camphor; triazine derivatives such as anisotriazine, ethylhexyl triazone, diethylhexyl butamido triazone, and 2,4,6-tris(diisobutyl 4'-aminobenzalmalonate)-s-triazine; phenylbenzimidazole derivatives such as phenylbenzimidazole sulfonic acid and disodium phenyldibenzimidazole tetrasulfonate; phenylbenzotriazol derivatives such as drometrizole trisiloxane and methylene bis-benzotriazolyl tetramethylbutylphenol; anthranilic derivatives such as menthyl anthranilate; imidazoline derivatives such as ethylhexyl dimethoxybenzylidene dioxoimidazoline propionate; benzalmalonate derivatives such as polyorganosiloxanes with benzalmalonate functional groups; and 4,4-diarylbutadiene derivatives such as 1,1-dicarboxy(2,2'-dimethylpropyl)-4,4-diphenylbutadiene.

UV scatters include such as zinc oxide, titanium dioxide. The surface-treated (hydrophobized) UV scatters are preferably used.

Examples of organic-modified clay minerals include quaternary ammonium cation modified clay minerals.

As the water-in-oil type emulsion sunscreen cosmetics of the present invention, there are milky liquid products and creamy products. These products can be prepared by the conventional method by mixing the above-described essential components and the components that are normally blended in cosmetics.

EXAMPLES

Hereinafter, the present invention will be described in further detail with reference to examples. However, the present invention is not limited by the following examples. All the blending amounts are shown in % by mass.

Before the examples are described, test methods and evaluation methods used in the present invention will be described.

[Example 1, Comparative examples 1 to 5, Comparative examples 6 to 13, and Examples 2 to 3]

A sunscreen cosmetic compositions were prepared according to the formulation shown in the following Tables 1 to 3.

In the formulation shown in Table 1, to be more precise, the sunscreen cosmetics were prepared by adding component (9) [= part B], to components (1) to (8) [= part A], to be dispersed homogeneously, and adding thereto components (10) to (12) [=part C], to be dispersed homogeneously, and then gradually adding thereto components (13) to (17) [=part D] to be emulsified.

In the formulation shown in Table 2, the sunscreen cosmetic was prepared by adding component (14) [= part B], to components (1) to (13) [= part A], to be dispersed homogeneously, and adding thereto components (15) to (17) [= part C], to be dispersed homogeneously, and then gradually adding thereto components (18) to (22) [= part D], to be emulsified.

In the formulation shown in Table 3, the sunscreen cosmetic was prepared by adding components (12) to (13) [= part B], to components (1) to (11) [= part A], to be dispersed homogeneously, and adding thereto components (14) to (16) [= part C], to be dispersed homogeneously, and then gradually adding thereto components (17) to (23) [= part D], to be emulsified.

The water resistance, the emulsion stability and the usability such as non-oily feeling and fresh light feeling of the respective obtained sunscreen cosmetics (samples) in Example 1, Comparative examples 1 to 5, Comparative examples 6 to 13, and Examples 2 to 3 were evaluated according to the following evaluation criteria. The results are shown in Tables 1 to 3.

[Water Resistance]

(Test Method)

The absorbance at a wavelength of 315 nm of the PMMA plate on which 2 mg/cm$^2$ of the respective examples were applied were measured both before and after soaking in running water for 30 minutes, and the value of a in the following formula was calculated.

$$a = \text{The absorbance after soaking in running water} \,/\, \text{The absorbance before soaking in running water}$$

(Evaluation Criteria)

○ : The value of a is 0.95 or more.
△ : The value of a is 0.8 or more and less than 0.95.

X : The value of a is 0.8 or less.

[Emulsion Stability]

(Test Method)

A half of the respective examples were filled in respective glass tubes and rotated at 45 rpm for 4 hours. The respective states were observed with eyes.

(Evaluation Criteria)

○ : No change in the state of the rotated example was observed compared with that of the pre-rotated example.
△ : Though no change in the appearance of the rotated example was observed with the eyes compared with that of the pre-rotated example, the emulsified particles in the rotated example became larger than that in the pre-rotated example when observing with a microscope.
X : Changes such as poor emulsification in the appearance of the rotated example was observed with the eyes compared with that of the pre-rotated example.

[Usability (oily feeling and stickiness)]

10 female panelists actually used respective samples and evaluated them according to the following evaluation criteria.

(Evaluation Criteria)

○ : Among 10 Panelists, 8 Panelists or more judged that it had good usability (non-oily feeling and non-stickiness).
△ : Among 10 Panelists, 5 to 8 Panelists judged that it had good usability (non-oily feeling and non-stickiness).
X : Among 10 Panelists, less than 5 Panelists judged that it had good usability (non-oily feeling and non-stickiness).

Table 1

| | Ex. 1 | Comp. Ex. 1 | Comp. Ex. 2 | Comp. Ex. 3 | Comp. Ex. 4 | Comp. Ex. 5 |
|---|---|---|---|---|---|---|
| (1) Decamethylcyclopentasiloxane | 19 | 11.5 | 5 | 23.99 | 9 | 21.99 |
| (2) Dimethylpolysiloxane (*1) [component(c)] | 5 | 5 | 5 | 0.01 | 15 | 5 |
| (3) Isononyl isononate [component(d)] | 3 | 3 | 3 | 3 | 3 | 0.01 |
| (4) Isostearic acid | 0.25 | 0.25 | 0.25 | 0.25 | 0.25 | 0.25 |
| (5) Trimethylsiloxysilicate | 2.5 | 2.5 | 2.5 | 2.5 | 2.5 | 2.5 |
| (6) Ethylhexyl methoxycinnamate (*2) [component(a)] | 7.5 | 15 | 7.5 | 7.5 | 7.5 | 7.5 |
| (7) Octocrylene (*3) [component (b)] | 5 | 5 | 15 | 5 | 5 | 5 |

(continued)

| | Ex. 1 | Comp. Ex. 1 | Comp. Ex. 2 | Comp. Ex. 3 | Comp. Ex. 4 | Comp. Ex. 5 |
|---|---|---|---|---|---|---|
| (8) Silicone-chain branched-type alkyl /POE-modified silicone (I) (*4) [component(e)] | 1 | 1 | 1 | 1 | 1 | 1 |
| (9) Organic-modified clay minerals (*5) | 0.2 | 0.2 | 0.2 | 0.2 | 0.2 | 0.2 |
| (10) Silicone-treated zinc oxide | 15 | 15 | 15 | 15 | 15 | 15 |
| (11) Fatty acid-treated titanium dioxide | 4 | 4 | 4 | 4 | 4 | 4 |
| (12) Polymethylsilsesquioxane | 6 | 6 | 6 | 6 | 6 | 6 |
| (13) Ion-exchanged water | 25.1 | 25.1 | 25.1 | 25.1 | 25.1 | 25.1 |
| (14) Glycerin | 1 | 1 | 1 | 1 | 1 | 1 |
| (15) Trisodium edetate | 0.1 | 0.1 | 0.1 | 0.1 | 0.1 | 0.1 |
| (16) Ethyl alcohol (95%) | 5 | 5 | 5 | 5 | 5 | 5 |
| (17) Phenoxyethanol | 0.35 | 0.35 | 0.35 | 0.35 | 0.35 | 0.35 |
| Water resistance | ○ | Δ | Δ | Δ | ○ | ○ |
| Emulsion stability | ○ | X | X | ○ | X | X |
| Usability (non-oily feeling and non-stickiness) | ○ | X | X | ○ | ○ | ○ |

Table 2

| | Comp. Ex. 6 | Comp. Ex. 7 | Comp. Ex. 8 | Comp. Ex. 9 | Comp. Ex. 10 | Comp. Ex. 11 | Comp. Ex. 12 | Comp. Ex. 13 |
|---|---|---|---|---|---|---|---|---|
| (1) Decamethylcyclopentasiloxane | 7 | 19 | 19 | 19.99 | 13 | 19 | 19 | 19 |
| (2) Dimethylpolysiloxane (*1) [component(c)] | 5 | 5 | 5 | 5 | 5 | 5 | 5 | 5 |
| (3) Isononyl isononate [component(d)] | 15 | - | - | 3 | 3 | 3 | 3 | 3 |
| (4) Caprylyl methicone | - | 3 | - | - | - | - | - | - |
| (5) Diisopropyl sebacate | - | - | 3 | - | - | - | - | - |
| (6) Isostearic acid | 0.25 | 0.25 | 0.25 | 0.25 | 0.25 | 0.25 | 0.25 | 0.25 |
| (7) Trimethylsiloxysilicate (8) | 2.5 | 2.5 | 2.5 | 2.5 | 2.5 | 2.5 | 2.5 | 2.5 |
| Ethylhexyl methoxycinnamate (*2) [component(a)] | 7.5 | 0.1 | 15 | 7.5 | 7.5 | 7.5 | 7.5 | 7.5 |
| (9) Octocrylene (*3) [component (b)] | 5 | 5 | 5 | 5 | 5 | 5 | 5 | 5 |
| (10) Silicone-chain branched-type alkyl/POE-modified silicone (1) (*4) [component(e)] | 1 | 1 | 1 | 0.01 | 7 | - | - | - |
| (11) POE-modified silicone (*6) | - | - | - | - | - | 1 | - | - |

(continued)

| | Comp. Ex. 6 | Comp. Ex. 7 | Comp. Ex. 8 | Comp. Ex. 9 | Comp. Ex. 10 | Comp. Ex. 11 | Comp. Ex. 12 | Comp. Ex. 13 |
|---|---|---|---|---|---|---|---|---|
| (12) Alkyl/POE-modified silicone (*7) | - | - | - | - | - | - | 1 | - |
| (13) Silicone-chain branched-type POE-modified silicone (*8) | - | - | - | - | - | - | - | 1 |
| (14) Organic-modified clay minerals (*5) | 0.2 | 0.2 | 0.2 | 0.2 | 0.2 | 0.2 | 0.2 | 0.2 |
| (15) Silicone-treated zinc oxide | 15 | 15 | 15 | 15 | 15 | 15 | 15 | 15 |
| (16) Fatty acid-treated titanium dioxide | 4 | 4 | 4 | 4 | 4 | 4 | 4 | 4 |
| (17) Polymethylsilsesquioxane | 6 | 6 | 6 | 6 | 6 | 6 | 6 | 6 |
| (18) Ion-exchanged water | 25.1 | 25.1 | 25.1 | 25.1 | 25.1 | 25.1 | 25.1 | 25.1 |
| (19) Glycerin | 1 | 1 | 1 | 1 | 1 | 1 | 1 | 1 |
| (20) Trisodium edetate | 0.1 | 0.1 | 0.1 | 0.1 | 0.1 | 0.1 | 0.1 | 0.1 |
| (21) Ethyl alcohol (95%) | 5 | 5 | 5 | 5 | 5 | 5 | 5 | 5 |
| (22) Phenoxyethanol | 0.35 | 0.35 | 0.35 | 0.35 | 0.35 | 0.35 | 0.35 | 0.35 |
| Water resistance | Δ | ○ | ○ | ○ | X | ○ | ○ | ○ |
| Emulsion stability | ○ | X | X | X | ○ | X | Δ | Δ |
| Usability (non-oily feeling and non-stickiness) | X | Δ | Δ | ○ | X | ○ | ○ | ○ |

Table 3

| | Ex. 2 | Ex. 3 |
|---|---|---|
| (1) Decamethylcyclopentasiloxane | 16.7 | 24.5 |
| (2) Dimethylpolysiloxane (*1) [component(c)] | 5 | - |
| (3) Dimethylpolysiloxane (*9) [component(c)] | - | 3 |
| (4) Isononyl isononate [component(d)] | 5 | - |
| (5) Diisopropyl sebacate | - | 5 |
| (6) Isostearic acid | 0.25 | 0.25 |
| (7) Trimethylsiloxysilicate | 2.5 | 2.5 |
| (8) Ethylhexyl methoxycinnamate (*2) [component(a)] | 2.5 | 5 |
| (9) Octocrylene (*3) [component(b)] | 8 | 2 |
| (10) Silicone-chain branched-type alkyl/POE-modified silicone (*4) [component(e)] | 1 | 1 |
| (11) Silicone-chain branched-type POE-modified silicone (*8) | 0.5 | - |
| (12) Organic-modified clay minerals (*5) | - | 0.2 |
| (13) Crosslinked-type polyether-modifeid silicone (*10) | 2 | - |
| (14) Silicone-treated zinc oxide | 15 | 15 |
| (15) Fatty acid-treated titanium dioxide | 4 | 4 |
| (16) Polymethylsilsesquioxane | 6 | 6 |

(continued)

| | Ex. 2 | Ex. 3 |
|---|---|---|
| (17) Ion-exchanged water | 25.1 | 20.3 |
| (18) Glycerin | 1 | 1 |
| (19) Trisodium edetate | 0.1 | 0.1 |
| (20) Ethyl alcohol (95%) | 5 | 5 |
| (21) Phenoxyethanol | 0.35 | 0.35 |
| (22) Triethanolamine | - | 1.8 |
| (23) Phenylbenzimidazole sulfonic acid (*11) | - | 3 |
| Water resistance | ○ | ○ |
| Emulsion stability | ○ | ○ |
| Usability (non-oily feeling and non-stickiness) | ○ | ○ |

In Tables 1 to 3, the following products were the compounds used in the examples of the present invention.

Dimethylpolysiloxane (*1) : "KF96A-6cs" manufactured by Shin-Etsu Chemical Co.,Ltd., ethylhexyl methoxycinnamate (*2) : "PARSOL MCX" manufactured by DSM Nutrition Japan K.K., octocrylene (*3): "PARSOL340" manufactured by DSM Nutrition Japan K.K., silicone-chain branched-type alkyl/POE-modified silicone (I) (*4) : "KF6038" manufactured by Shin-Etsu Chemical Co.,Ltd., organic-modified clay minerals (*5) : "Bentone 38V CG" manufactured by Elementis Specialties, Inc., POE-modified silicone (*6) : "KF6013" manufactured by Shin-Etsu Chemical Co.,Ltd., alkyl/POE-modified silicone (*7) : "Abil EM90" manufactured by TH Goldschmidt Ltd., silicone-chain branched-type POE-modified silicone (*8) : "KF 6028" manufactured by Shin-Etsu Chemical Co.,Ltd., dimethylpolysiloxane (*9) : "KF96A-5000cs" manufactured by Shin-Etsu Chemical Co.,Ltd., crosslinked-type polyether-modifeid silicone (*10) : "KSG 210" manufactured by Shin-Etsu Chemical Co.,Ltd., phenylbenzimidazole sulfonic acid (*11) : "Eusolex232" manufactured by Merck & Co., Inc.

As is clear from the result in Tables 1 to 3, the water-in-oil type emulsion sunscreen cosmetics of the present invention containing components (a) to (e) was confirmed to be excellent in all of the UV protection capability (sunscreen effect), the water resistance, the emulsion stability, and the usability such as non-oily feeling and non-stickiness.

## Claims

1. A water-in-oil type emulsion sunscreen cosmetic comprising (a) 0.2 to 14 % by mass of methoxycinnamate ester, (b) 0.02 to 14 % by mass of octocrylene, (c) 0.2 to 14 % by mass of dimethylpolysiloxane, (d) 0.02 to 14 % by mass of monoester oil represented by the formula: $R_1COOR_2$ wherein $R_1$ represents an alkyl group having 5 to 11 carbon atoms and $R_2$ represents an alkyl group having 3 to 11 carbon atoms, and (e) 0.02 to 6 % by mass of silicone-chain branched-type alkyl/polyoxyethylene-modified silicone.

2. The water-in-oil type emulsion sunscreen cosmetic according to claim 1 wherein the component (a) is ethylhexyl methoxycinnamate.

3. The water-in-oil type emulsion sunscreen cosmetic according to claim 1 or2 wherein the component (d) is an ester formed by isocarboxylic acid and isoalcohol and each $R_1$ and $R_2$ have 7 to 10 carbon atoms.

## INTERNATIONAL SEARCH REPORT

International application No.

PCT/JP2007/051787

### A. CLASSIFICATION OF SUBJECT MATTER

*A61K8/37*(2006.01)i, *A61K8/06*(2006.01)i, *A61K8/40*(2006.01)i, *A61K8/891*
(2006.01)i, *A61K8/894*(2006.01)i, *A61Q17/04*(2006.01)i

According to International Patent Classification (IPC) or to both national classification and IPC

### B. FIELDS SEARCHED

Minimum documentation searched (classification system followed by classification symbols)
A61K8/37, A61K8/06, A61K8/40, A61K8/891, A61K8/894, A61Q17/04

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2005-206573 A  (Nippon Fine Chemical Co., Ltd.), 04 August, 2005 (04.08.05), Par. No. [0151]; example 117 (Family: none) | 1-3 |
| Y | JP 2001-039819 A  (Shin-Etsu Chemical Co., Ltd.), 13 February, 2001 (13.02.01), Claims; Par. Nos. [0003] to [0004], [0007] & DE 60001693 T2          & EP 1072627 A2 & JP 3724988 B2          & KR 20010015478 A & US 6576623 B1 | 1-3 |

☒ Further documents are listed in the continuation of Box C.          ☐ See patent family annex.

| | |
|---|---|
| * Special categories of cited documents:<br>"A" document defining the general state of the art which is not considered   to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 24 April, 2007 (24.04.07) | 15 May, 2007 (15.05.07) |

| Name and mailing address of the ISA/<br>Japanese Patent Office | Authorized officer |
|---|---|
| Facsimile No. | Telephone No. |

Form PCT/ISA/210 (second sheet) (April 2005)

| | | |
|---|---|---|
| **INTERNATIONAL SEARCH REPORT** | International application No. | |
| | PCT/JP2007/051787 | |

C (Continuation).　DOCUMENTS CONSIDERED TO BE RELEVANT

| Category* | Citation of document, with indication, where appropriate, of the relevant passages | Relevant to claim No. |
|---|---|---|
| Y | JP 2004-161698 A (Kose Corp.),<br>10 June, 2004 (10.06.04),<br>Claims 1 to 5; Par. Nos. [0005], [0007], [0021]<br>& CN 1504180 A　　　　& HK 1065259 A1<br>& KR 20040042876 A　　& US 2004/137025 A1 | 1-3 |
| A | WO 2003/028690 A1 (BEIERSDORF AG.),<br>10 April, 2003 (10.04.03),<br>Example 80<br>& DE 10148301 A1　　　& DE 10148302 A1<br>& DE 10148313 A1　　　& DE 10148314 A1<br>& DE 10150619 A1　　　& DE 10155960 A1<br>& EP 1434562 A1　　　& JP 2005/508919 A<br>& US 2004/258721 A1 | 1-3 |
| E,A | WO 2007/017921 A1 (Shiseido Co., Ltd.),<br>15 May, 2007 (15.05.07),<br>Example 2<br>(Family: none) | 1-3 |

Form PCT/ISA/210 (continuation of second sheet) (April 2005)

| INTERNATIONAL SEARCH REPORT | International application No. |
|---|---|
| | PCT/JP2007/051787 |

The range of "a branched-silicone-chain type alkyl- and polyoxyethylene-modified silicone" set forth in claim 1 is unclear, though the whole of the description has been examined. Thus, claim 1 does not satisfy the requirement of clarity as provided for in PCT Article 6.

Form PCT/ISA/210 (extra sheet) (April 2005)

**EP 1 992 328 A1**

## REFERENCES CITED IN THE DESCRIPTION

**Patent documents cited in the description**

- JP 2006028874 A

- JP H9151108 B